# FASCICULE DE BREVET EUROPEEN

(11) **EP 2 235 030 B1**
(45) Date de publication et mention de la délivrance du brevet: **12.08.2015**
(21) Numéro de dépôt: 08872171.7
(22) Date de dépôt: 27.11.2008
(51) Int. Cl.: C07H 3/04, C07H 15/18, A61K 31/7016

(54) **COMPOSÉ DU TYPE DISACCHARIDE, COMPOSITION CONTENANT UN TEL COMPOSÉ ET PROCÉDÉ DE FABRICATION D'UN TEL COMPOSÉ**
DISACCHARID-DERIVAT, EINE SOLCHE VERBINDUNG ENTHALTENDE ZUSAMMENSETZUNG UND EIN VERFAHREN ZUR HERSTELLUNG SOLCHER VERBINDUNGEN
COMPOUND OF THE DISACCHARIDE TYPE COMPOSITION COMPRISING AND METHOD FOR PRODUCING SUCH A COMPOUND

(30) Priorité: 28.11.2007 FR 0708313
(43) Date de publication de la demande: 06.10.2010
(73) Titulaire: Université de Picardie Jules Verne, 80025 Amiens (FR)
(72) Inventeur: BULTEL, Laurent, F-80000 Amiens (FR); SAGUEZ, Julien, F-60120 Bonneuil les Eaux (FR); GIORDANENGO, Philippe, F-80800 Marcelcave (FR); KOVENSKY, José, F-80000 Amiens (FR)
(74) Mandataire: Bertrand, Didier
(86) Numéro de dépôt international: PCT/FR2008/001653
(87) Numéro de publication internationale: WO 2009/098400

(56) Documents cités:
- EP-A- 0 497 313
- LA FERLA ET AL: "Synthesis of disaccharidic sub-units of a new series of heparin related oligosaccharides" TETRAHEDRON, ELSEVIER SCIENCE PUBLISHERS, AMSTERDAM, NL, vol. 55, no. 32, 6 août 1999 (1999-08-06), pages 9867-9880, XP005393171 ISSN: 0040-4020
- UDODONG U E ET AL: "A ready, convergent synthesis of the heptasaccharide GPI membrane anchor of rat brain Thy-1 glycoprotein" JOURNAL OF THE AMERICAN CHEMICAL SOCIETY, AMERICAN CHEMICAL SOCIETY, WASHINGTON, DC.; US, US, vol. 115, 1 janvier 1993 (1993-01-01), pages 7886-7887, XP002155268 ISSN: 0002-7863
- HOUSTON D R ET AL: "Structure-based exploration of cyclic dipeptide chitinase inhibitors" JOURNAL OF MEDICINAL CHEMISTRY, USAMERICAN CHEMICAL SOCIETY. WASHINGTON, vol. 47, no. 23, 1 janvier 2004 (2004-01-01), pages 5713-5720, XP003014252 ISSN: 0022-2623
- KATO T ET AL: "Styloguanidines, New Chitinase Inhibitors from the Marine Sponge Stylotella Aurantium" TETRAHEDRON LETTERS, ELSEVIER, AMSTERDAM, vol. 36, no. 12, 20 mars 1995 (1995-03-20), pages 2133-2136, XP004028424 ISSN: 0040-4039
- RAO ET AL: "Methylxanthine Drugs Are Chitinase Inhibitors: Investigation of Inhibition and Binding Modes" CHEMISTRY AND BIOLOGY, CURRENT BIOLOGY, LONDON, GB, vol. 12, no. 9, 1 septembre 2005 (2005-09-01), pages 973-980, XP005088924 ISSN: 1074-5521
- USUKI HIROKAZU ET AL: "Screening and partial characterization of inhibitors of insect beta-N-acetylglucosaminidase" JOURNAL OF PESTICIDE SCIENCE, vol. 31, no. 1, 2006, pages 41-46, XP002489864 ISSN: 1348-589X cité dans la demande

## Description

La présente invention concerne un composé du type disaccharide, un procédé d'obtention d'un tel composé ainsi qu'une composition contenant un tel composé.

La chitine est une substance glucidique (polymère de N-acétylglucosamine) qui est présente en grande quantité dans la nature et forme, notamment, le squelette externe des insectes. Les insectes grandissant par mues successives, ils secrètent des enzymes, appelées chitinases, qui dégradent la chitine. Ces sont ces enzymes qui leur permettent de muer et de grandir. Il existe plusieurs catégories de chitinases. On trouve des chitinases dans les organismes des insectes, des animaux marins et chez certains végétaux ou champignons. On sait que l'inhibition des chitinases bloque la croissance des insectes, notamment, en empêchant ou freinant leurs mues.

Parmi les molécules qui peuvent être considérées comme des inhibiteurs de chitinases, les oligo- ou polysaccharides sont de très bons candidats en raison de leur mimétisme avec la chitine. C'est pourquoi plusieurs molécules ont été testées sur les insectes.

Le chitosan est un polysaccharide biodégradable, obtenu par *N-dé* acétylation de la chitine. Il est employé pour diverses applications biotechnologiques et ses propriétés insecticides sont rapportées sur les lépidoptères *Spodoptera littoralis, Helicoverpa armigera* et *Plutella xylostella,* mais aussi sur les pucerons *Aphis gossypii, Metopolophium dirhodum, Hyalopterus pruni, Rhopalosiphum padi, Sitobion avenae* et *Myzus persicae.* L'alimentation de ces insectes avec le chitosan induit un développement larvaire anormal, une inhibition de la prise alimentaire, des mues interrompues et surtout une augmentation de la mortalité (confère l'article de Rabea EI, Badawy MEI, Rogge TM, Stevens CV, Steurbaut W, Höfte M et Smagghe G (2006) « Enhancement of fungicidal and insecticidal activity by reductive alkylation of chitosan ». Pest Manag Sci 62: 890-897). Toutefois, les cibles et mécanismes d'actions de ce composé ne sont pas connus et le chitosan n'est pas rapporté comme étant un inhibiteur de chitinases. Cependant, l'origine et les homologies de structure de ce composé avec la chitine laissent supposer que le chitosan pourrait être un substrat des chitinases.

L'inhibiteur de chitinases le plus étudié est l'allosamidine, un pseudotrisaccharide décrit par Sakuda S, Isogai A, Matsumoto S et Suzuki A (1987), dans l'article « Search for microbial insect growth regulators. II. Allosamidin, a novel insect chitinase inhibitor » publié dans J Antibiot 40: 296-300. Constituée de deux groupements *N*-acétylallosamine liés à une allosamizoline, la molécule d'allosamidine présente une structure analogue à celle de la chitine mais n'est pas hydrolysable (voir l'article de Bortone K, Monzingo AF, Ernst S et Robertus JD (2002), intitulé « The structure of an allosamidin complex with the Coccidioides immitis chitinase defines a role for a second acid residue in substrate-assisted mechanism » et publié dans J Mol Biol 320: 293-302). Isolé à partir de bactéries du genre *Steptomyces,* l'allosamidine exerce une puissante activité inhibitrice contre les chitinases d'insectes et de champignons (confère l'article de Blattner R, Gerard P et Spindler-Barth M, intitulé « Synthesis and biological activity of allosamidin and allosamidin derivatives » et publié dans Pestic Sci 50: 312-318). En agissant par inhibition compétitive, ce composé présente de nombreux effets délétères sur la mue chez le lépidoptère *Bombyx mori* et la mouche *Lucilia cuprina*. Généralement, la perturbation des mues s'accompagne de retards de croissance et de forts taux de mortalité comme chez la mite du vêtement *Tineola bisselliella.*

L'allosamidine possède également des effets multiples dans l'interaction entre le plasmodium responsable du paludisme et son hôte, le moustique *Aedes aegypti.* Les chitinases du plasmodium et du moustique sont inhibées (voir l'article de Tsai YL, Hayward RE, Langer RC, Fidock DA et Vinetz JM (2001), intitulé « Disruption of Plasmodium falciparum chitinase markedly impairs parasite invasion of mosquito midgut » et publié dans Infect Immun 69: 4048-4054), induisant des modifications dans l'organisation, la perméabilité et la dégradation de la membrane péritrophique (voir l'article de Shahabuddin M, Toyoshima T, Aikawa M et Kaslow DC (1993), intitulé « Transmission-blocking activity of a chitinase inhibitor and activation of malarial parasite chitinase by mosquito protease » et publié dans Proc Natl Acad Sci USA 90: 4266-4270).

Des ôligo- et polysaccharides, isolés à partir de filtrats de cultures de champignons mycéliens, présentent des activités inhibitrices contre des chitinases de *Spodoptera litura* mais pas contre celles de *Streptomyces griseus.* Parmi les polysaccharides isolés de ces cultures fongiques, l'un d'eux, a été isolé de la souche TNPT116-Cz de *Sphaeropsis sp.* Ce polysaccharide nommé FPS-1 serait composé de deux résidus N-acétylglucosamine, d'un glucose et d'un galactose. Le FPS-1 inhibe fortement la chitinase de *S*. *litura* avec un pouvoir inhibiteur comparable à celui de l'allosamidine (voir l'article de Nitoda T, Usuki H, Kurata A et Kanzaki H (2003) intitulé « Macromolecular insect chitinase inhibitors produced by fungi: screening and partial characterization » et publié dans J Pestic Sci 28:24-32).

De plus, La Ferla et al. (Tetrahedron, 1999, vol. 55, no. 32,), Udodong et al. (J. Am. Chem. Soc.1993, vol. 115), le document de brevet EP-A-0497313, Houston et al. (J. Med. Chem., 2004, vol. 47, no. 23), Kato et al. (Tetrahedron, 1995, vol. 36, no. 12,), Rao et al. (Chem and biol, 2005, vol. 12, no.9) décrivent divers procédés de synthèse.

Les trois composés précités présentent au moins un résidu *N-*acétylglucosamine, lié à une unité glucosamine par une liaison β-1,4 glycosidique ce qui rend leur structure proche de celle de la chitine.

Un but de la présente invention est de proposer une nouvelle famille de composés qui présentent des propriétés d'inhibition des chitinases.

Ce but est atteint au moyen d'un composé chimique du type disaccharide et présentant la formule générale (A) suivante : 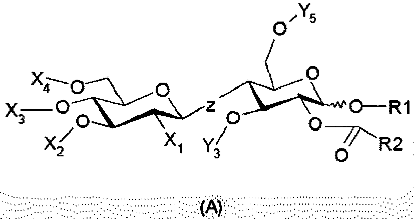 dans laquelle,
Z représente un atome O ou S en position β;
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ , un radical -CHϕ formant un cycle avec l'atome d'oxygène en C(5), dans lesquels ϕ représente un radical phényle ; un atome H ou un radical sulfonate ;
X₄ représente un atome H, un radical sulfonate ou l'atome de carbone du radical - CHϕ représenté par X₃;
R₁ représente un radical allyle contenant de 2 à 5 atomes de carbone ou un radical alkyl contenant de 1 à 5 atomes de carbone, la liaison OR₁ étant en position α ou en β;
R₂ représente un radical alkyl contenant de 1 à 5 atomes de carbone ;
Y₃ représente un radical benzyle, un atome H ou un radical sulfonate ;
Y₅ représente un atome H ou un radical sulfonate.

Lorsque Z représente un atome d'oxygène en position β, on retrouve bien l'analogie de structure avec la chitine. Lorsque Z représente un atome de soufre, le composé de formule générale (A) représente donc un S-disaccharide. Ce composé S-disaccharide, bien que présentant une structure plus éloignée de celle de la chitine, réagit néanmoins avec les chitinases et forme avec elles des liaisons plus stables.

Par ailleurs, la Demanderesse a également constaté que la présence de radicaux sulfonate en tant que substituant X2 et/ou X3 et/ou X4 et/ouY3 et/ou Y5 permet d'accélérer la cinétique de la réaction du composé de l'invention avec les chitinases. De ce fait, le composé de l'invention réagit plus vite avec les chitinases que la chitine et se substitue donc à cette dernière, inhibant les effets de chitinases.

Selon un mode de réalisation particulier, Z représente un atome O en position β;
X₁ représente un radical azido ;
X₂ représente un radical benzyle ;
X₃ représente un radical -CHϕ formant un cycle l'atome d'oxygène en C(5), dans lequel ϕ représente un radical phényle ;
X₄ représente l'atome de carbone du radical -CHϕ représenté par X₃;
R₁ représente un radical allyle contenant de 2 à 5 atomes de carbone, la liaison OR₁ étant en position α ou β;
R₂ représente un radical méthyle ;
Y₃ représente un radical benzyle ;
Y₅ représente un atome d'hydrogène.

Selon un autre second mode de réalisation,
Z représente un atome O en position β;
X₁ représente un radical NHCOR₃ dans lequel R₃ est un radical méthyle ;
X₂ représente un radical benzyle ;
X₃ représente un radical -CHϕ formant un cycle avec l'atome d'oxygène en C(5), dans lequel ϕ représente un radical phényle ;
X₄ représente l'atome de carbone du radical-CHϕ représenté par X₃ ;
R₁ représente un radical allyle contenant de 2 à 5 atomes de carbone, la liaison OR₁ étant en α ou en β;
R₂ représente un radical méthyle ;
Y₃ représente un radical benzyle ;
Y₅ représente un atome d'hydrogène.

Selon un troisième mode de réalisation,
Z représente un atome O en position β;
X₁ représente un radical NHCOR₃ dans lequel R₃ est un radical méthyle ;
X₂ représente un radical benzyle ;
X₃ représente un radical -CHϕ formant un cycle avec l'atome d'oxygène en C₅, dans lesquels ϕ représente un radical phényle ;
X₄ représente l'atome de carbone du radical-CHϕ représenté par X₃ ;
R₁ représente un radical allyle contenant de 2 à 5 atomes de carbone, la liaison OR₁ étant en α ou en β;
R₂ représente un radical méthyle ;
Y₃ représente un radical benzyle ;
Y₅ représente un radical sulfonate.

Selon un quatrième mode de réalisation,
Z représente un atome O en position β;
X₁ représente un radical NHCOR₃ dans lequel R₃ est un radical méthyle ;
X₂ représente un atome H ;
X₃ représente un atome H ;
X₄ représente un atome H ;
R₁ représente un radical propyle, la liaison OR₁ étant en α ou en β;
R₂ représente un radical méthyle ;
Y₃ représente un atome H ;
Y₅ représente un atome H.

Selon un cinquième mode de réalisation,
Z représente un atome O en position β;
X₁ représente un radical NHCOR₃ dans lequel R₃ est un radical méthyle ;
X₂ représente un atome H ;
X₃ représente un atome H ;
X₄ représente un atome H ;
R₁ représente un radical propyle, la liaison OR₁ étant en position α ou β;
R₂ représente un radical méthyle ;
Y₃ représente un atome H ;
Y₅ représente un radical sulfonate.

La présente invention concerne également une composition comprenant au moins un composé selon l'invention, utilisée en tant que médicament, agent prophylactique ou agent phytosanitaire.

En effet, comme tous les inhibiteurs de chitinases, les composés de l'invention présentent des propriétés antifongiques et antibactériennes. Dans ce cas, le ou les composés de l'invention sont mélangés à un excipient ou à tout autre vecteur facilitant son utilisation et son action.

La composition comprenant au moins un composé de formule générale (A) peut être utilisée en tant qu'inhibiteur de chitinases, ce qui lui confère des propriétés en tant qu'insecticide, notamment.

Ainsi, la composition comprenant au moins un composé de formule générale (A) peut être utilisée en tant qu'insecticide.

Avantageusement, la composition, lorsqu'elle est utilisée en tant qu'insecticide, contient une concentration dudit ou desdits composés comprise entre 10 et 100 µg/ml, et de préférence, entre 10 et 50 µg/ml.

La présente invention concerne également un procédé de préparation d'un composé présentant la formule générale (A) 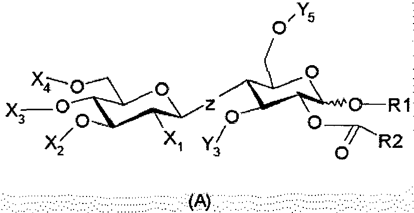 dans laquelle, Z représente un atome O ou S en position β ;
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ, un radical -CHϕ formant un cycle avec l'atome d'oxygène en C(5), dans lesquels ϕ représente un radical phényle ; un atome H ou un radical sulfonate ;
X₄ représente un atome H, un radical sulfonate ou l'atome de carbone du radical - CHϕ représenté par X₃;
R₁ représente un radical allyle contenant de 2 à 5 atomes de carbone ou un radical alkyl contenant de 1 à 5 atomes de carbone;
R₂ représente un radical alkyl contenant de 1 à 5 atomes de carbone ;
Y₃ représente un radical benzyle, un atome H ou un radical sulfonate ;
Y₅ représente un atome H ou un radical sulfonate.
selon lequel :
- on fait réagir un composé de formule générale (B) 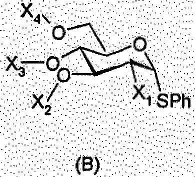
dans laquelle
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ , un radical -CHϕ formant un cycle avec l'atome d'oxygène en C(5), dans lesquels ϕ représente un radical phényle ; un atome H ou un radical sulfonate ;
X₄ représente un atome H, un radical sulfonate ou l'atome de carbone du radical - CHϕ représenté par X₃;
Ph représente un radical phényle ;
avec un composé de formule générale (C) 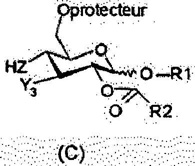 dans laquelle
Z représente un atome O ou S ;
R₁ représente un radical allyle contenant de 2 à 5 atomes de carbone ;
R₂ représente un radical alkyl contenant de 1 à 5 atomes de carbone ;
Y₃ représente un radical benzyle, un atome d'hydrogène ou un radical sulfonate ;
« Protecteur » représente un groupe protecteur ;
pour obtenir un composé de formule générale (D) 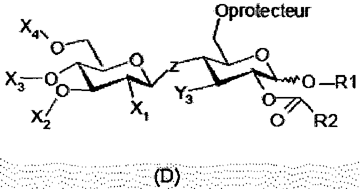 dans laquelle
Z représente un atome O ou S en position β;
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ ou un radical -CHϕ formant un cycle avec l'atome d'oxygène en C(5), dans lesquels ϕ représente un radical phényle ;
X₄ représente un atome d'hydrogène, un radical sulfonate ou l'atome de carbone du radical -CHϕ représenté par X₃;
R₁ représente un radical allyle en α ou β et contenant de 2 à 5 atomes de carbone ; R₂ représente un radical alkyl contenant de 1 à 5 atomes de carbone ;
Y₃ représente un radical benzyle, un atome d'hydrogène ou un radical sulfonate ;
« Protecteur » représente un groupe protecteur ;
   - on fait réagir ledit composé (D) présentant une liaison β(1, 4) glycosique de manière à substituer le groupement protecteur par un atome H ou un radical sulfonate pour obtenir ledit composé de formule générale (A).

Le groupe O-protecteur peut être un groupe TBDMSO qui sera retiré par réaction avec du tétrahydrofurane sec et du tétrabutylammonium (TBAF). Le groupement O-protecteur peut également être un groupement TBSO.

La réaction de glycosylation entre le composé de formule générale (B) et le composé de formule générale (C) peut être optimisée pour l'obtention du disaccharide β. Il existe de nombreuses méthodes pour le faire, que ce soit par un changement de conditions de réaction, par un changement de groupement activateur de la position anomère (trichloroacétimidate dans l'acétonitrile), ou par un changement du groupement « protecteur », qui est dans le cas présent le groupement COR₃, sur l'azote (trichloroacétate, phthalimidate).

Lorsque le groupement X1 est un groupement phthalimido, c'est-à-dire lorsque X1= N(CO)2Ph, on obtient exclusivement le disaccharide BETA.

La présente invention, ses caractéristiques et les différents avantages qu'elle procure seront mieux compris à la lecture de la description qui suit d'un exemple de synthèse d'un composé selon l'invention, et de l'exposé des résultats obtenus lors de l'utilisation de ce composé sur des pucerons.

Par souci de simplification, le composé selon l'invention dont la synthèse va être décrite ci-dessous est dénommé DPS2. La description qui suit s'intéresse à ce composé particulier, mais il est bien évident que tout autre composé selon l'invention présente les mêmes propriétés.

### Synthèse du DPS2

Ce procédé décrit la préparation de deux nouvelles molécules actives I et II (composés 6 et 8) à partir de précurseurs connus.

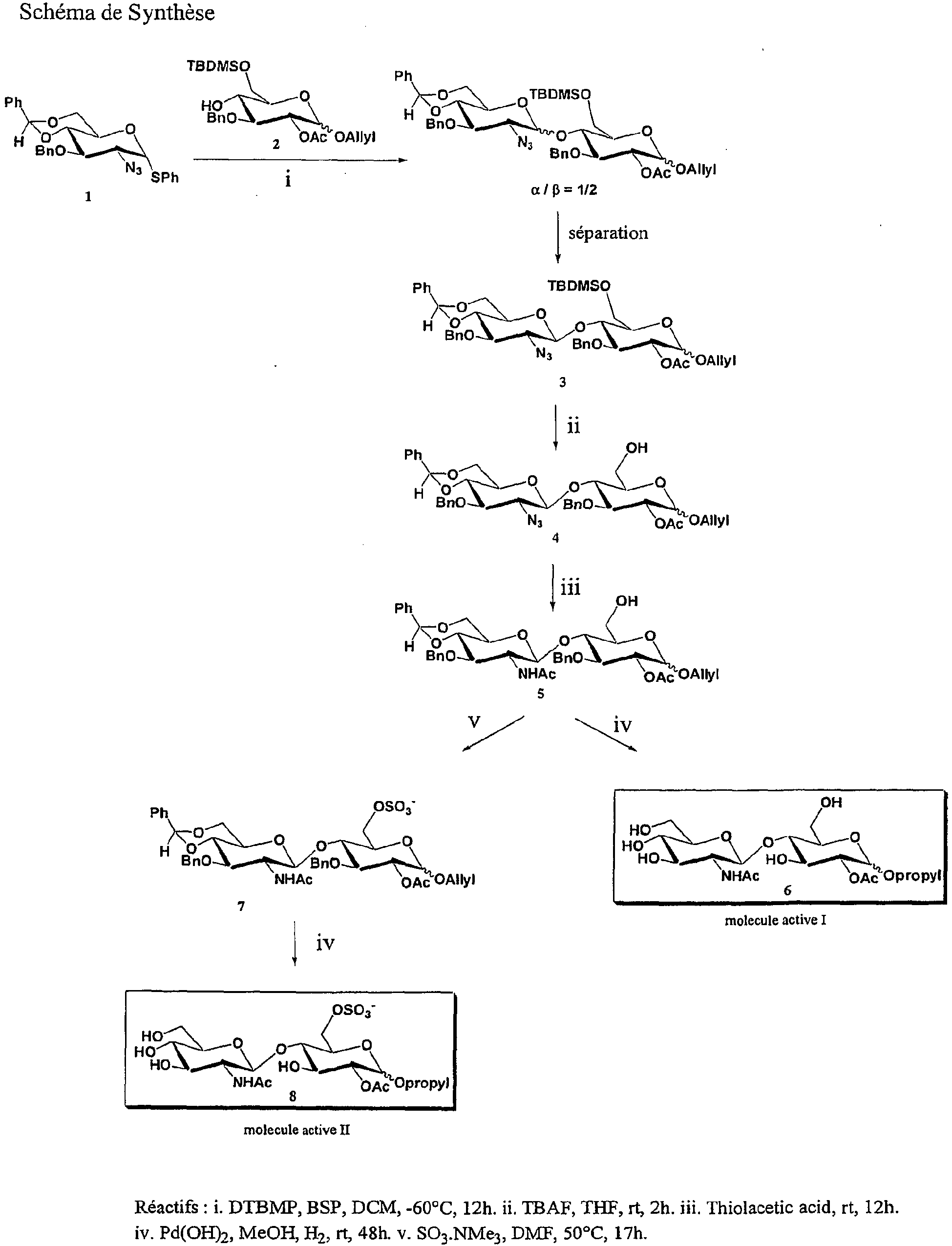

La molécule 1 a déjà été décrite [Lindberg, Jan; Ohberg, Liselotte; Garegg, Per J.; Konradsson, Peter dans "Efficient routes to glucosamine-myo-inositol derivatives, key building blocks in the synthesis of glycosylphosphatidylinositol anchor substances" Tetrahedron (2002), 58(7), 1387-1398].

La molécule 2 est nouvelle et a été préparée à partir d'un précurseur connu, le 2-O-acétyl-3-O-benzyl-D-glucopyranoside d'allyle [Martin-Lomas, Manuel; Flores-Mosquera, Maria; Chiara, Jose Luis. « Attempted synthesis of type-A inositolphosphoglycan mediators - synthesis of a pseudo-hexasaccharide precursor »Eur. J.Org. Chem. (2000), (8), 1547-1562].

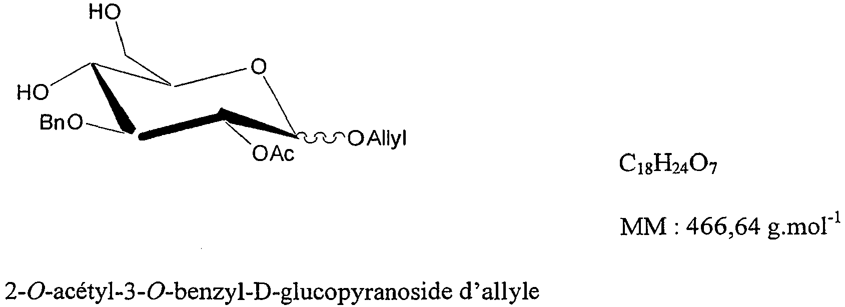

### Préparation du 2-O-acétyl-3-O-benzyl-6-O-t-butyldiméthylsilyl-D-glucopyranoside d'allyle (2)

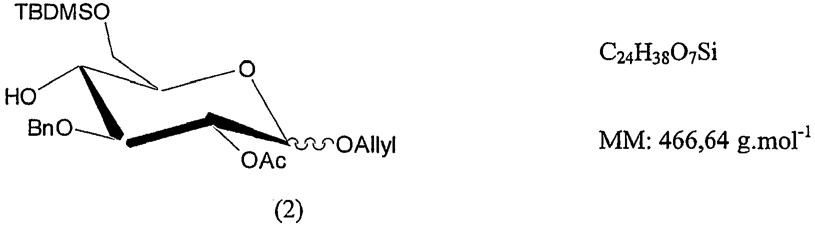

Au 2-*O*-acétyl-3-*O*-benzyl-D-glucopyranoside d'allyle (9 g, 25,57 mmol) en solution dans du dichlorométhane sec (80 mL), on ajoute le chlorure de *tert-*butyldiméthylsilyle (TBDMSCl) (5,78 g, 38,3 mmol, 1,5 éq.), la 4-diméthylaminopyridine (DMAP) (150 mg, 1,27 mmol, 0,05 éq.) et enfin de la triéthylamine (TEA) (6 mL, 43,5 mmol, 1,7 éq.). Le milieu réactionnel est laissé sous agitation pendant 4 h à température ambiante, puis dilué avec du dichlorométhane (100 mL). La phase organique est lavée successivement avec une solution saturée d'hydrogénocarbonate de sodium (NaHCO₃) (2 x 100 mL) et une solution saturée de chlorure de sodium (NaCl) (2 x 100 mL), séchée sur sulfate de magnésium anhydre (MgSO₄), filtrée et concentrée sous vide. Le brut est ensuite chromatographié sur gel de silice avec comme éluant 20/80 AcOEt/cyclohexane pour conduire au composé 2 (10 g, 21,46 mmol, 84%).
Eluant du suivi de réaction par C.C.M. : 50/50 AcOEt/cyclohexane => Rf = 0.81

### Préparation du 2-azido-3-O-benzyl-4,6-O-benzylidène-2-désoxy-D-glucopyranosyl-β-(1→4)-2-O-acétyl-3-O-benzyl-6-O-tert-butyldiméthylsityl-D-glucopyranoside d'allyle (3)

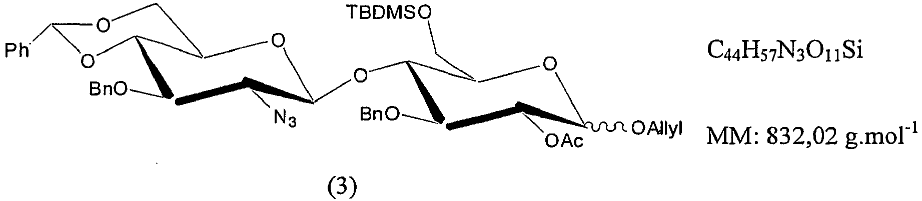

A du dichlorométhane sec (2 mL) on additionne de la 1-(phénylsulfinyl)-pipéridine (BSP) (64 mg, 0,299 mmol, 1,3 éq.), de la 2, *6-tert*-butyl-4-méthylpyridine (DTBMP) (62 mg, 0,299 mmol, 1,3 éq.) et du tamis moléculaire 4 Å (400 mg) le tout est agité à température ambiante sous atmosphère inerte (argon) pendant 30 minutes.

Le milieu est ensuite refroidit à -60°C. On additionne ensuite de l'anhydride trifluorométhane sulfonique (Tf₂O) (52 µL, 0,299 mmol, 1,3 éq.), suivi, 30 minutes après, de l'addition d'une solution du composé 1 (111 mg, 0,23 mmol) dans du dichlorométhane sec (2 mL) par canule. Le tout est laissé sous agitation a -60°C sous argon pendant 15 minutes. Enfin on additionne une solution du composé 2 (218 mg, 0,46 mmol, 2 éq.) dans du dichlorométhane sec (2 mL) là encore à l'aide d'une canule.

Le milieu réactionnel est lentement réchauffé jusqu'à 10°C sur une période de 12 h. La réaction est ensuite neutralisée par addition de méthanol (10 mL), le tout est filtré sur cartouche de célite, puis concentré sous pression réduite. Le brut est ensuite chromatographié sur gel de silice avec comme éluant 1/99 AcOEt/toluène pour conduire au composé 3 alpha (27,4 mg, 0,033 mmol) et 2/98 AcOEt/toluène pour conduire au composé 3 bêta (74,8 Mg, 0,090 mmol).
Rendement : 60%
Eluant du suivi de réaction par C.C.M. : 20/80 AcOEt/toluène => Rf = 0.66 pour l'alpha, Rf = 0.60 pour le bêta.

### Préparation du 2-azido-3-O-benzyl-4,6-O-benzylidène-2-désoxy-D-glucopyranosyl-β-(1→4)-2-O-acétyl-3-O-benzyl-D-glucopyranoside d'allyle (4)

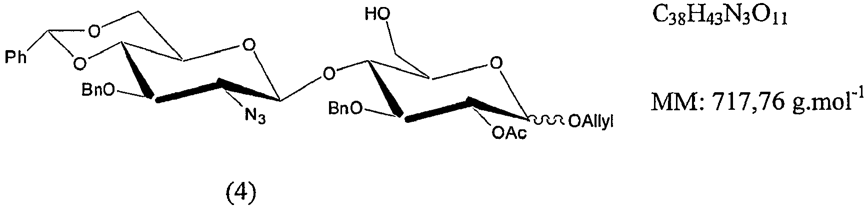

Au composé 3 bêta (34,7 mg, 0,0417 mmol) sous argon, on additionne du tétrahydrofurane sec (THF) (2 mL). Le tout est agité et refroidit à 0°C à l'aide d'un bain de glace. On ajoute ensuite du fluorure de tétrabutylammonium (TBAF) (19,7 mg, 0,062 mmol, 1,5 éq.).

Le milieu réactionnel est agité sous argon à température ambiante sur une période de 2h.

La réaction est stoppée par addition de *N,N-*diisopropyléthylamine (1 mL), puis le THF est évaporé sous pression réduite. Le brut réactionnel et ensuite repris dans de l'eau (10 mL) puis est extrait avec du dichlorométhane (2 X 10 mL). La phase organique est séchée sur sulfate de magnésium anhydre (MgSO₄), filtrée et évaporée sous pression réduite. Le brut est alors chromatographié sur gel de silice avec comme éluant 30/70 AcOEt/cyclohexane pour conduire au composé 4 (24,3 mg, 0,0338 mmol, 81%).
Eluant du suivi de réaction par C.C.M. : 10/90 AcOEt/toluène => Rf = 0.13.

### Préparation du 2-acétamido-3-O-benzyl-4,6-O-benzylidène-2-désoxy-D-glucopyranosyl-β-(1→4)-2-O-acétyl-3-O-benzyl-D-glucopyranoside d'allyle (5)

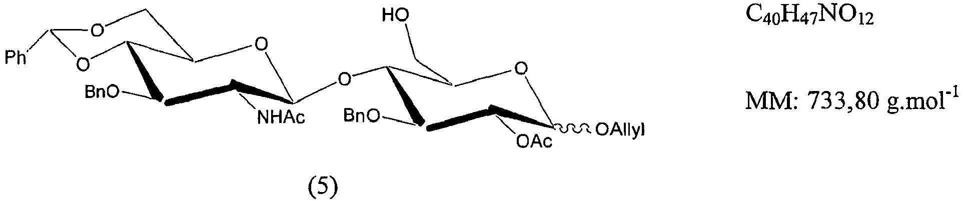

Au composé 4 (26,5 mg, 0,0369 mmol) en solution dans du diméthyleformamide (DMF) (2 mL) on additionne de l'acide thioacétique (0,3 mL). Le tout est laissé sous agitation à température ambiante pendant 24h. Le solvant est évaporé sous pression réduite, puis le brut est dilué avec du dichlorométhane (10 mL). La phase organique est lavée avec de l'eau (2 x 10 mL), séchée sur sulfate de magnésium anhydre (MgSO₄), filtrée et concentrée sous vide. Le brut est ensuite chromatographié sur gel de silice avec comme éluant 70/30 AcOEt/cyclohexane pour conduire au composé 5 (11,7 mg, 0,016 mmol, 43%).
Eluant du suivi de réaction par C.C.M. : 60/40 AcOEt/cyclohexane => Rf = 0.57

### Préparation du -2-acétamido-2-désoxy-D-glucopyranosyl-β-(1→4)-2-O-acétyl D-glucopyranoside de propyle (6)

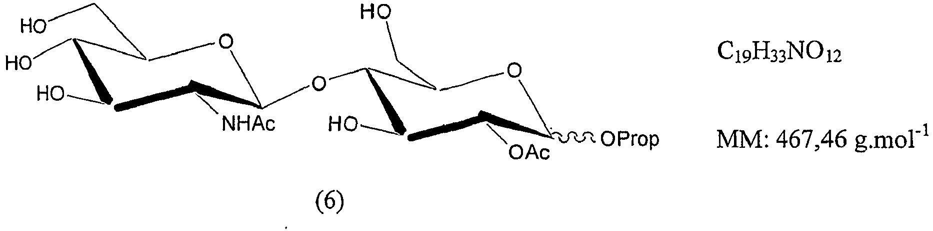

11,7 g (0,016 mmol) de composé 5 sont solubilisé dans 2 mL de MeOH et 2 mg de Pd(OH)2 (10% massique) sont ajoutés. Le milieu est ensuite placé sous hydrogène. Après 12h, le milieu réactionnel est filtré et le solvant est éliminé sous pression réduite pour conduire à 7,8 g (Rdt = quantitatif) de composé 6.

### Préparation du 2-acétamido-3-O-benzyl-4,6-D-benzylidène-2-désoxy-D-glucopyranosyl-β-(1→β)-2-O-acétyl-3-O-benzyl-6-O-sulfonate-D-glucopyranoside d'allyle (7)

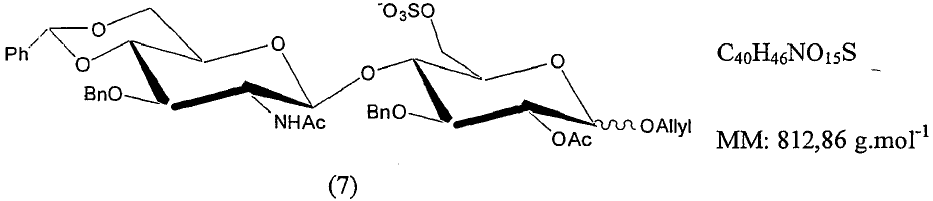

A une solution de composé 5 (20 mg, 0,027 mmol) dans du diméthylformamide (DMF) (5 mL) on additionne des sels de sulfate de triméthylammonium (SO₃Me₃N) (75,9 mg, 0,545 mmol, 20 éq.). Le milieu réactionnel est placé sous agitation, sous atmosphère inerte (argon), à 55°C, pendant 18h.

Le DMF est évaporé sous pression réduite et le brut est purifié à deux reprises sur résine de type séphadex avec comme éluant le mélange méthanol / dichlorométhane (1 :1, V : V). Les fractions contenant le composé sont réunies et le solvant évaporé. Rendement: quantitatif.

### Préparation du 2-acétamido-2-désoxy-D-glucopyranosyl-(3-(1→4)-2-O-acétyl-6-O-sulfonate-D-glucopyranoside de propyle (8) (DPS2)

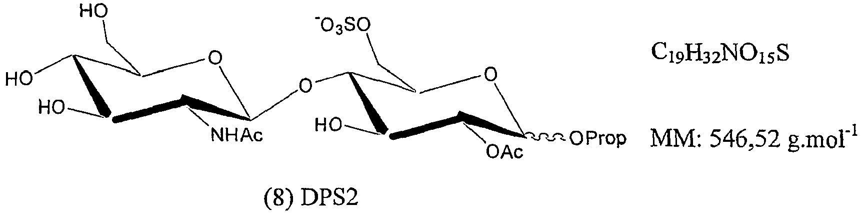

50 mg (0,062 mmol) de composé 7 sont solubilisés dans 5 mL de MeOH et 5 mg de Pd(OH)₂ (10% massique) sont ajoutés. Le milieu est ensuite placé sous hydrogène. Après 48 h, le milieu réactionnel est filtré et le solvant est éliminé sous pression réduite pour conduire à 30 mg (rendement : quantitatif) de composé 8 (DPS2).

Par simple modification du composé de formule générale (B), il est possible de synthétiser un précurseur thiol du composé de l'invention. Ceci peut se faire par double substitution (en passant par un triflate, par exemple, suivi d'une double inversion) ou par une réaction de Mitsunobu. Ce précurseur sera glycosylé par le même donneur permettant l'obtention de l'analogue soufré, plus stable en milieu biologique vis-à-vis des enzymes.

### Utilisation du DPS2

Deux espèces de pucerons *Myzus persicae* (Sulzer) et *Macrosiphum euphorbiae* (Thomas) ont été utilisées pour cette étude. Des adultes ont été isolés et placés dans des cages de PVC sur milieu artificiel pendant une journée afin de synchroniser la descendance. Les néonates, âgés de moins de 24 h sont alors placés sur milieu artificiel contenant des concentrations variant de 0 à 100 µg.mL⁻¹ du composé DPS2, synthétisé précédemment, selon un protocole décrit par Saguez J, Dubois F, Vincent C, Laberche JC, Sangwan-Norreel BS et Giordanengo P (2006), dans l'article intitulé « Differential aphicidal effects of chitinase inhibitors on the polyphagous homopteran Myzus pensicae (Sulzer) » et publié dans Pest Manag Sci 62: 1150-1154. Plusieurs paramètres démographiques sont relevés quotidiennement afin de déterminer la durée du développement larvaire, la mortalité larvaire et la fécondité et de calculer un paramètre synthétique, le rₘ (taux intrinsèque d'accroissement des populations) et le temps de doublement des populations. Un minimum de 50 pucerons de chaque espèce est testé par traitement et les individus sont suivis quotidiennement. La taille des pucerons a été mesurée une semaine après le début de l'expérience, sous loupe binoculaire à l'aide d'un oculaire biométrique. Les effets du DPS2 sur la survie larvaire sont analysés par un test du χ² de Pearson. Une analyse de variance (ANOVA monofactorielle) a été réalisée avec le logiciel Statistica 5.5 (StatSoft®, Tulsa, Oklahoma, USA) pour tester les effets du DPS2 sur les paramètres démographiques des aphides. Les paramètres significativement altérés sont ensuite analysés par un test de Fisher (PLSD) au seuil de significativité P <0,05.

### Résultats

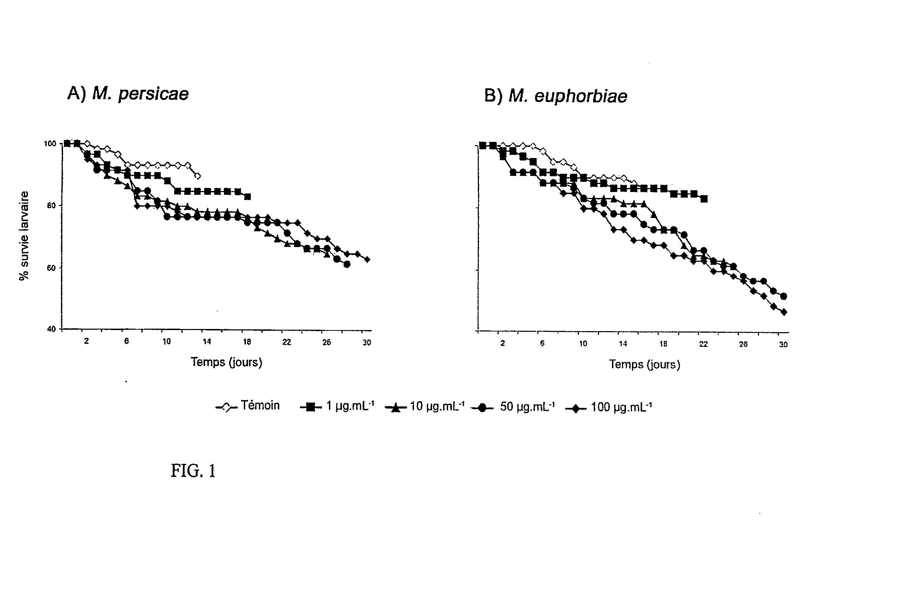

La figure 1 représente la mortalité larvaire des pucerons *M. persicae* et *M. euphorbiae* élevés sur milieux artificiels complémentés en disaccharide sulfaté à des concentrations variant de 0 à 100 µg.mL⁻¹.

Les résultats montrent des effets délétères du DPS2 sur les deux espèces de pucerons, avec des effets plus marqués chez *M. euphorbiae* que sur *M. persicae.* Quelle que soit l'espèce de puceron, la survie larvaire est significativement affectée par cette molécule. On observe ainsi une réduction de la survie larvaire d'environ 35 à 40% aux concentrations de 10 à 100µg.mL⁻¹ chez *M. pensicae (χ²* = 89,19 dl=30 p<0,00 ; χ² = 94,90 dl=30 p<0,00 ; χ² = 77,82 dl=30 p<0,00) alors que chez *M. euphorbiae,* la mortalité larvaire atteint 49 à 53% aux concentrations respectives de 50 et 100 µg.mL⁻¹ (χ² = 108,06 dl=30 p<0,00 ; χ² = 151,97 dl=30 p<0,00). De plus, de nombreux individus n'atteignent pas l'état adulte et meurent après un mois de traitement, sans avoir donné de descendance alors que sur un milieu témoin, l'ensemble des individus devient adulte en deux semaines.

Les processus de mue sont affectés lorsque la concentration en DPS2 est comprise entre 50 et 100 µg.mL⁻¹, Ainsi, de nombreux aphides n'arrivent pas à terminer leur mue et meurent avant même d'avoir pu s'extraire de leur ancienne cuticule. La taille des pucerons est réduite chez *M*. *euphorbiae* à partir de 10 µg.mL⁻¹. Quel que soit le paramètre étudié et l'espèce de puceron considérée, les effets aphicides maximum sont obtenus dès la concentration de 50 µg.mL⁻¹. Les effets sont similaires aux concentrations de 50 et 100 µg.mL⁻¹.

Dans les tableaux I, II et III qui suivent, les résultats sont exprimés sous la forme : moyenne ± écart-type. L'effet significatif (*) été testé par une analyse de variance monofactorielle (F) à P<0.05. Les valeurs de même rang suivies par une même lettre indiquent qu'il n'existe pas de différence significative d'après le test du PLSD de Fisher (P<0.05).

La durée de la période pré-reproductive est également affectée par un apport en DPS2 dans l'alimentation des pucerons, puisqu'elle est augmentée d'environ une journée chez *M. persicae* et de deux jours aux plus fortes concentrations chez *M. euphorbiae.* Les paramètres démographiques des adultes (fécondité, période d'oviposition, fécondité journalière, taux intrinsèque d'accroissement naturel et temps de doublement des populations) sont également modifiés lorsque les pucerons sont alimentés sur un milieu complémenté en DPS2, et les effets sont marqués pour toutes les concentrations. Ainsi, la fécondité journalière est réduite de 10 à 35% sur les milieux contenant de 10 à 100 µg.mL⁻¹ de DPS2 et de façon dose-dépendante. La valeur du taux intrinsèque d'accroissement des populations (*rₘ*) qui tient compte des paramètres précédents est également réduite chez les deux espèces d'aphides, y compris pour la plus faible concentration de 1 µg.mL⁻¹. Concernant le temps de doublement des populations, qui dépend directement du rₘ, il est significativement augmenté d'environ 1,5 à 2 jours pour les concentrations les plus élevées chez *M. persicae* et de près de 4 jours chez *M. euphorbiae.* L'ensemble de ces résultats nous permet donc de conclure que cet oligosaccharide induit des effets aphicides très marqués sur les deux espèces de pucerons *M. persicae* et *M. euphorbiae*.

Le DPS2 présente des effets délétères sur pucerons en perturbant à la fois le développement larvaire et les processus de reproduction. Certains aphides meurent avant même d'avoir pu effectuer complètement leur mue. Les aphides survivants possèdent également une taille inférieure après une semaine d'alimentation plusieurs d'entre eux ne produisent pas de descendance. Or, la taille des pucerons est un paramètre important dans la reproduction puisque des pucerons de taille réduite sont généralement moins féconds. Ces différences de taille pourraient être à l'origine de l'absence ou de la diminution de fécondité. L'absence de descendance et la réduction de fécondité observées pourraient également être dues à l'inhibition des chitinases nécessaires au remodelage des structures tégumentaires lors du développement embryonnaire des pucerons.

Bien qu'il ne soit pas possible, aujourd'hui, de confirmer que les chitinases sont les cibles de cette molécule, la Demanderesse estime, sans être pour autant liée à cette explication, que, puisque la molécule de DPS2 présente des homologies de structures avec la chitine et les inhibiteurs de chitinase d'origine polysaccharidique et qu'elle induit des effets comparables, on peut supposer que le DPS2 ainsi que tout autre composé de l'invention, se lie directement au site actif des chitinases d'insectes et qu'il exerce une fonction inhibitrice sur leur métabolisme.

Les molécules de l'invention peuvent également avoir d'autres effets sur d'autres enzymes cibles telles que des glucose hydrolases ou des glycosidases. En effet, les polysaccharides sont souvent décrits dans l'inhibition d'endoglycosidases. Ainsi, les amylostatines et les liposaccharides bactériens inhibent respectivement les β-glucosidases et les lysozymes. Les chitinases appartenant à la même famille enzymatique que les sucrases, impliquées dans la dégradation du saccharose et dans les processus d'osmorégulation, on peut envisager un spectre d'action plus large des composés de l'invention.

Les composés obtenus selon l'invention offrent de nouvelles perspectives dans le développement de méthodes de lutte alternatives contre les pucerons, les insectes en général mais aussi tous les organismes constitués de chitine ou utilisant des chitinases pour leur développement. En effet, les propriétés antibactériennes, antifongiques et insecticides des inhibiteurs de chitinases et des composés décrits précédemment ont souvent été rapportées. Le chitosan présente de multiples propriétés parmi lesquelles des effets antifongiques contre les champignons pathogènes des cultures, *Botrytis cinerea* et *Pyricularia grisea.* Des propriétés insecticides sont également rapportées sur les lépidoptères *Spodoptera littoralis*, *Helicoverpa armigera* et *Plutella xylostella,* mais aussi les pucerons *Aphis gossypii, Metopolophium dirhodum*, *Hyalopterus pruni*, *Rhopalosiphum padi, Sitobion avenae* et *Myzus persicae* (voir l'article de Rabea EI, Bdawy MEI, Rogge TM, Stevens CV, Steurbaut W, Höfte Met Smagghe G (2006) intitulé « enhancement of fungicidal and insecticidal activity by reductive alkylation of chitosan », publié dans Pest Manag Sci 62: 890-897). De même, les effets de l'allosamidine et de ses dérivés ne se limitent pas uniquement aux insectes. En effet, ces inhibiteurs retardent l'enkystement d'amibes (voir l'article de Villagomez-Castro JC, Calvo-Mendez C et Lopez-Romero E (1992), intitulé « Chitinase activity in encysting Entamoeba invadens and its inhibition by allosamidin » et publié dans Mol Biochem Parasitol 52: 53-62) et inhibent les chitinases de nématodes et celles de bactéries. L'allosamidine possède également des propriétés antifongiques (voir l'article de MacNab R. et Glover L.A. (1991) intitulé « inhibition of Neurospora crassa cytosolic chitinase by albumin », publié dans FEMS Microbiol Lett 82 : 79-82). Il est donc fort probable que la famille de composés de la présente invention présente les mêmes propriétés que les inhibiteurs de chitinases connus.

L'ensemble de ces données souligne l'intérêt d'une stratégie anti-chitinase dans un contexte de phytoprotection. Les propriétés aphicides décrites avec le DPS2, confirment l'importance du mimétisme des composés saccharidiques dans la lutte contre les insectes et nous avons ouvert de nouvelles voies de recherches pour l'élaboration de nouveaux composés phytosanitaires.

Les groupements substitués sur des composés de l'invention, peuvent selon leur nature, modifier la structure et l'encombrement stérique des molécules de l'invention et donc modifier la nature et la force des interactions entre la molécule de l'invention et l'enzyme. Les molécules de l'invention présentent des propriétés aphicides mais ont potentiellement des effets antifongiques et antibiotiques. Elles peuvent occuper une place de choix dans des programmes de lutte intégrée contre les pathogènes et ravageurs des cultures.

**Tableau I : Effets du DPS2 sur la taille de M. euphorbiae alimenté sur milieu artificiel à des concentrations variant de 0 à 100 µg.mL⁻¹ après 7 jours de traitement**

| concentration en DPS2 (µg.mL-1) | témoin | 1 | 10 | 50 | 100 | F | P |
|---|---|---|---|---|---|---|---|
| nombre de pucerons | 37 | 33 | 33 | 32 | 33 | | |
| taille des pucerons (mm) | 2,3 ± 0,3^{a} | 2,2 ± 0,3^{a} | 2,1 ± 0,3^{b} | 1,8 ± 0,2^{bc} | 1,7 ± 0,2^{c} | 37,6 | <0,00* |

**Tableau II : Effets sur M. persicae alimentés sur un milieu artificiel à des concentrations en DPS2 variant de 0 à 100 µg.mL⁻¹**

| Concentration en DPS2 (µg.mL⁻¹) | Témoin | 1 | 10 | 50 | 100 | F | P |
|---|---|---|---|---|---|---|---|
| Nombre de pucerons | 54 | 50 | 39 | 37 | 38 | | |
| Période préreproductive (jours) | 10,6 ± 1,4^{a} | 11,6 ± 1,9^{b} | 10,9 ± 1,5^{a} | 11,1 ± 1,4^{a} | 11,9 ± 1,7^{b} | 4,5 | <0,00* |
| Fécondité journalière (larves.femelles-1.jour-1 | 1,07 ± 0,28^{a} | 0,99 ± 0,27^{a} | 0,91 ± 0,28^{b} | 0,74 ± 0,24^{bc} | 0,65 ± 0,11^{bc} | 20,9 | <0,00* |
| rₘ (femelles.femelles⁻¹ .jours⁻¹) | 0,16 ± 0,05^{a} | 0,13 ± 0,04^{b} | 0,13 ± 0,03^{b} | 0,12 ± 0,03^{bc} | 0,11 ± 0,03^{c} | 11,2 | <0,00* |
| Temps de doublement de la population (jours) | 4,4 ± 0,9^{a} | 5,6 ± 1,8^{b} | 5,4 ± 1,2^{b} b | 5,9 ± 1,6^{b} b | 6,5 ± 2,0^{a} | 10,9 | <0,00* |

**Tableau III : Effets sur M. euphorbiae alimentés sur un milieu artificiel à des concentrations variant de 0 à 100 µg.mL⁻¹**

| Concentration en DPS2 (µg.mL⁻¹) | Témoin | 1 | 10 | 50 | 100 | F | P |
|---|---|---|---|---|---|---|---|
| Nombre de pucerons | 52 | 50 | 37 | 31 | 28 | | |
| Période préreproductive (jours) | 11,6 ± 2,1^{a} | 11,3 ± 2,1^{a} | 12,1 ± 1,6^{a} | 13,4 ± 1,2^{b} | 13,5 ± 1,6^{b} | 10,8 | <0,00* |
| Fécondité journalière (larves.femelles-1 .jour-1 | 1,13 ± 0,36^{a} | 1,16 ± 0,36^{a} | 1,06 ± 0,34^{a} | 0,81 ± 0,20^{b} | 0,73 ± 0,22^{b} | 13,3 | <0,00* |
| rₘ (femelles.femelles⁻¹.jours⁻¹) | 0,16 ± 0,05^{a} | 0,16 ± 0,04^{b} | 0,13 ± 0,03^{b} | 0,09 ± 0,03^{c} | 0,08 ± 0,03^{c} | 52,3 | <0,00* |
| Temps de doublement de la population (jours) | 4,4 ± 0,9^{a} | 4,7 ± 1,0^{ab} | 5,5 ± 1,3^{b} | 8,1 ± 1,6^{c} | 6,5 ± 2,0^{c} | 58,8 | <0,00* |

## Revendications

1. Composé chimique du type disaccharide et présentant la formule générale (A) suivante : 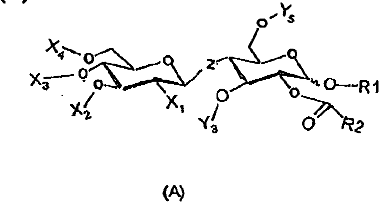 dans laquelle,
Z représente un atome O ou S en position β;
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ, un radical -CHϕ formant un cycle avec l'atome d'oxygène en C(5), dans lesquels ϕ représente un radical phényle ; un atome H ou un radical sulfonate ;
X₄ représente un atome H, un radical sulfonate ou l'atome de carbone du radical - CHϕ représenté par X₃;
R₁ représente un radical allyle contenant de 2 à 5 atomes de carbone ou un radical alkyl contenant de 1 à 5 atomes de carbone, la liaison OR₁ étant en α ou en β;
R₂ représente un radical alkyl contenant de 1 à 5 atomes de carbone ;
Y₃ représente un radical benzyle, un atome H ou un radical sulfonate ;
Y₅ représente un atome H ou un radical sulfonate.

2. Composé chimique selon la revendication 1, **caractérisé en ce que** :
Z représente un atome O en position β;
X₁ représente un radical azido ;
X₂ représente un radical benzyle ;
X₃ représente un radical -CHϕ formant un cycle l'atome d'oxygène en C(5), dans lequel ϕ représente un radical phényle ;
X₄ représente l'atome de carbone du radical -CHϕ représenté par X₃;
R₁ représente un radical allyle contenant de 2 à 5 atomes de carbone, la liaison OR₁ étant en position α ou β;
R₂ représente un radical méthyle ;
Y₃ représente un radical benzyle ;
Y₅ représente un atome d'hydrogène.

3. Composé chimique selon la revendication 1, **caractérisé en ce que** :
Z représente un atome O en position β;
X₁ représente un radical NHCOR₃ dans lequel R₃ est un radical méthyle ;
X₂ représente un radical benzyle ;
X₃ représente un radical -CH_{ϕ} formant un cycle avec l'atome d'oxygène en C(5),
dans lequel ϕ représente un radical phényle ;
X₄ représente l'atome de carbone du radical-CHϕ représenté par X₃ ;
R₁ représente un radical allyle contenant de 2 à 5 atomes de carbone, la liaison
OR₁ étant en position α ou β;
R₂ représente un radical méthyle ;
Y₃ représente un radical benzyle ;
Y₅ représente un atome d'hydrogène.

4. Composé chimique selon la revendication 1, **caractérisé en ce que** :
Z représente un atome O en position β;
X₁ représente un radical NHCOR₃ dans lequel R₃ est un radical méthyle ;
X₂ représente un radical benzyle ;
X₃ représente un radical -CHϕ formant un cycle avec l'atome d'oxygène en G₅,
dans lesquels ϕ représente un radical phényle ;
X₄ représente l'atome de carbone du radical-CHϕ représenté par X₃ ;
R₁ représente un radical allyle contenant de 2 à 5 atomes de carbone, la liaison OR₁ étant en position α ou β;
R₂ représente un radical méthyle ;
Y₃ représente un radical benzyle ;
Y₅ représente un radical sulfonate.

5. Composé chimique selon la revendication 1, **caractérisé en ce que** :
Z représente un atome O en position β;
X₁ représente un radical NHCOR₃ dans lequel R₃ est un radical méthyle ;
X₂ représente un atome H ;
X₃ représente un atome H ;
X₄ représente un atome H ;
R₁ représente un radical propyle, la liaison OR₁ étant en α ou en β ;
R₂ représente un radical méthyle ;
Y₃ représente un atome H ;
Y₅ représente un atome H.

6. Composé chimique selon la revendication 1, **caractérisé en ce que** :
Z représente un atome O en position β;
X₁ représente un radical NHCOR₃ dans lequel R₃ est un radical méthyle ;
X₂ représente un atome H ;
X₃ représente un atome H ;
X₄ représente un atome H ;
R₁ représente un radical propyle, la liaison OR₁ étant en α ou en β ;
R₂ représente un radical méthyle ;
Y₃ représente un atome H ;
Y₅ représente un radical sulfonate.

7. Composition comprenant au moins un composé selon l'une quelconque des revendications 1 à 6, utilisée en tant que médicament, agent prophylactique ou agent phytosanitaire.

8. Composition comprenant au moins un composé selon l'une quelconque des revendications 1 à 6, utilisée en tant qu'inhibiteur de chitinases.

9. Composition comprenant au moins un composé selon l'une quelconque des revendications 1 à 6, utilisée en tant qu'insecticide.

10. Composition selon l'une quelconque des revendications 7 à 9, **caractérisée en ce qu'**elle contient une concentration dudit ou desdits composés comprise entre 10 et 100 µg/ml et de préférence, entre 10 et 50 µg/ml.

11. Procédé de préparation d'un composé présentant la formule générale (A) 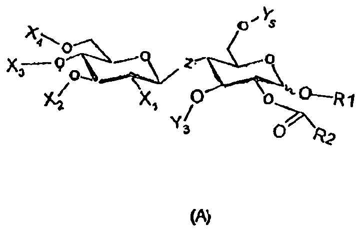 dans laquelle, Z représente un atome O ou S en positon
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ, un radical -CHϕ formant un cycle avec l'atome d'oxygène en C(5), dans lesquels ϕ représente un radical phényle ; un atome H ou un radical sulfonate ;
X₄ représente un atome H, un radical sulfonate ou l'atome de carbone du radical - CHϕ représenté par X₃;
R₁ représente un radical allyle contenant de 2 à 5 atomes de carbone ou un radical alkyl contenant de 1 à 5 atomes de carbone;
R₂ représente un radical alkyl contenant de 1 à 5 atomes de carbone ;
Y₃ représente un radical benzyle, un atome H ou un radical sulfonate ;
Y₅ représente un atome H ou un radical sulfonate.
selon lequel :
- on fait réagir un composé de formule générale (B) 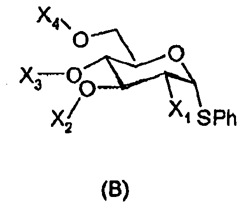
dans laquelle
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ, un radical -CHϕ formant un cycle avec l'atome d'oxygène en C(5), dans lesquels ϕ représente un radical phényle ; un atome H ou un radical sulfonate ;
X₄ représente un atome H, un radical sulfonate ou l'atome de carbone du radical - CHϕ représenté par X₃;
Ph représente un radical phényle ;
avec un composé de formule générale (C) 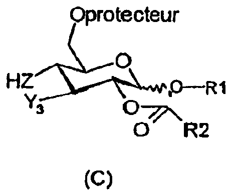 dans laquelle
Z représente un atome O ou S ;
R₁ représente un radical allyle contenant de 2 à 5 atomes de carbone ;
R₂ représente un radical alkyl contenant de 1 à 5 atomes de carbone ;
Y₃ représente un radical benzyle, un atome d'hydrogène ou un radical sulfonate ; Protecteur représente un groupe protecteur ;
pour obtenir un composé de formule générale (D) 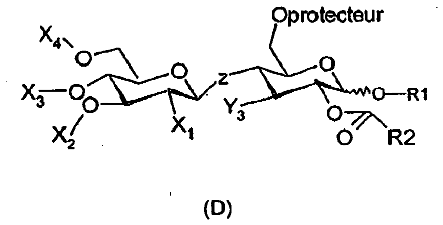 dans laquelle
Z représente un atome O ou S en position β;
X₁ représente un radical azido ou un radical NHCOR₃ dans lequel R₃ est un radical alkyl ou aryle contenant de 1 à 5 atomes de carbone ;
X₂ représente un radical benzyle, un atome H ou un radical sulfonate ;
X₃ représente un radical -CH₂ϕ ou un radical -CHϕ formant un cycle avec l'atome d'oxygène en C(5), dans lesquels ϕ représente un radical phényle ;
X₄ représente un atome d'hydrogène, un radical sulfonate ou l'atome de carbone du radical -CHϕ représenté par X₃;
R₁ représente un radical allyle en α ou β et contenant de 2 à 5 atomes de carbone ;
R₂ représente un radical alkyl contenant de 1 à 5 atomes de carbone ;
Y₃ représente un radical benzyle, un atome d'hydrogène ou un radical sulfonate ; Protecteur représente un groupe protecteur ;
- on fait réagir ledit composé (D) présentant une liaison β(1, 4) glycosique de manière à substituer le groupement protecteur par un atome H ou un radical sulfonate pour obtenir ledit composé de formule générale (A).

## Patentansprüche

1. Chemische Verbindung des Disaccharid-Typs, welche die folgende allgemeine Formel (A) aufweist: 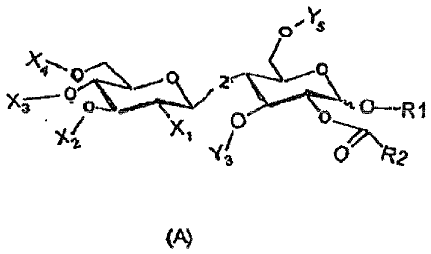 worin
Z ein β-ständiges O- oder S-Atom darstellt;
X₁ einen Azidrest oder einen NHCOR₃-Rest, worin R₃ ein Alkyl- oder Arylrest mit 1 bis 5 Kohlenstoffatomen ist, darstellt;
X₂ einen Benzylrest, ein H-Atom oder einen Sulfonatrest darstellt;
X₃ einen -CH₂ϕ-Rest, einen -CHϕ-Rest, der mit dem an C(5) gebundenen Sauerstoffatom einen Ring bildet, worin ϕ eines, Phenylrest darstellt; ein H-Atom oder einen Sulfonatrest darstellt;
X₄ ein H-Atom, einen Sulfonatrest oder das Kohlenstoffatom des -CHϕ-Restes, dargestellt durch X₃, darstellt;
R₁ einen Allylrest mit 2 bis 5 Kohlenstoffatomen oder einen Alkylrest mit 1 bis 5 Kohlenstoffatomen darstellt, wobei die Bindung OR₁ α- oder β-ständig ist;
R₂ einen Alkylrest mit 1 bis 5 Kohlenstoffatomen darstellt;
Y₃ einen Benzylrest, ein H-Atom oder einen Sulfonatrest darstellt;
Y₅ ein H-Atom oder einen Sulfonatrest darstellt.

2. Chemische Verbindung nach Anspruch 1, **dadurch gekennzeichnet, dass**:
Z ein β-ständiges O-Atom darstellt;
X₁ einen Azidrest darstellt;
X₂ einen Benzylrest darstellt;
X₃ einen -CHϕ-Rest darstellt, der mit dem an C(5) gebundenen Sauerstoffatom einen Ring bildet, worin ϕ einen Phenylrest darstellt;
X₄ das Kohlenstoffatom des -CHϕ-Restes, dargestellt durch X₃, darstellt;
R₁ einen Allylrest mit 2 bis 5 Kohlenstoffatomen darstellt, wobei die Bindung OR₁ α- oder β-ständig ist;
R₂ einen Methylrest darstellt,;
Y₃ einen Benzylrest darstellt;
Y₅ ein Wasserstoffatom dar stellt.

3. Chemische Verbindung nach Anspruch 1, **dadurch gekennzeichnet, dass**
Z ein β-ständiges O-Atom darstellt;
X₁ einen NHCOR₃-Rest, worin R₃ ein Methylrest ist, darstellt;
X₂ einen Benzylrest darstellt;
X₃ einen -CHϕ-Rest darstellt, der mit dem an C(5) gebundenen Sauerstoffatom einen Ring bildet, worin ϕ einen Phenylrest darstellt;
X₄ das Kohlenstoffatom des -CHϕ-Restes, dargestellt durch X₃, darstellt;
R₁ einen Allylrest mit 2 bis 5 Kohlenstoffatomen darstellt, wobei die Bindung OR₁ α- oder β-ständig ist;
R₂ einen Methylrest darstellt,
Y₃ einen Benzylrest darstellt;
Y₅ ein Wasserstoffatom darstellt.

4. Chemische Verbindung nach Anspruch 1, **dadurch gekennzeichnet, dass**:
Z ein β-ständiges O-Atom darstellt;
X₁ einen NHCOR₃-Rest, worin R₃ ein Methylrest ist, darstellt;
X₂ einen Benzylrest darstellt;
X₃ einen -CHϕ-Rest darstellt, der mit dem an C₅ gebundenen Sauerstoffatom einen Ring bildet, worin ϕ einen Phenylrest darstellt;
X₄ das Kohlenstoffatom des -CHϕ-Restes, dargestellt durch X₃, darstellt;
R₁ einen Allylrest mit 2 bis 5 Kohlenstoffatomen darstellt, wobei die Bindung OR₁ α- oder β-ständig ist;
R₂ einen Methylrest darstellt;
Y₃ einen Benzylrest darstellt;
Y₅ einen Sulfonatrest darstellt.

5. Chemische Verbindung nach Anspruch 1, **dadurch gekennzeichnet, dass**:
Z ein β-ständiges O-Atom darstellt;
X₁ einen NHCOR₃-Rest, worin R₃ ein Methylrest ist, darstellt;
X₂ ein H-Atom darstellt;
X₃ ein H-Atom darstellt;
X₄ ein H-Atom darstellt;
R₁ einen Propylrest darstellt, wobei die Bindung OR₁ α- oder β-ständig ist;
R₂ einen Methylrest darstellt;
Y₃ ein H-Atom darstellt;
Y₅ ein H-Atom darstellt.

6. Chemische Verbindung nach Anspruch 1, **dadurch gekennzeichnet, dass**:
Z ein β-ständiges O-Atom darstellt;
X₁ einen NHCOR₃-Rest, worin R₃ ein Methylrest ist, darstellt;
X₂ ein H-Atom darstellt;
X₃ ein H-Atom darstellt;
X₄ ein H-Atom darstellt;
R₁ einen Propylrest darstellt, wobei die Bindung OR₁ α- oder β-ständig ist;
R₂ einen Methylrest darstellt;
Y₃ ein H-Atom darstellt;
Y₅ einen Sulfonatrest darstellt.

7. Zusammensetzung, umfassend mindestens eine Verbindung nach einem der Ansprüche 1 bis 6, verwendet als Medikament, prophylaktisches Mittel oder phytosanitäres Mittel.

8. Zusammensetzung, umfassend mindestens eine Verbindung nach einem der Ansprüche 1 bis 6, verwendet als Chitinase-Inhibitor.

9. Zusammensetzung, umfassend mindestens eine Verbindung nach einem der Ansprüche 1 bis 6, verwendet als Insektizid.

10. Zusammensetzung nach einem der Ansprüche 7 bis 9, **dadurch gekennzeichnet, dass** sie die Verbindung oder die Verbindungen in einer Konzentration von 10 bis 100 µg/mL und vorzugsweise von 10 bis 50 µg/mL, enthält.

11. Verfahren zur Herstellung einer Verbindung, welche die allgemeine Formel (A) aufweist, 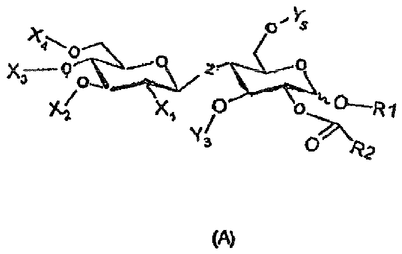
worin Z ein β-ständiges O- oder S-Atom darstellt;
X₁ einen Azidrest oder einen NHCOR₃-Rest, worin R₃ ein Alkyl- oder Arylrest mit 1 bis 5 Kohlenstoffatomen ist, darstellt;
X₂ einen Benzylrest, ein H-Atom oder einen Sulfonatrest darstellt;
X₃ einen -CH₂ϕ-Rest, einen -CHϕ-Rest, der mit dem an C(5) gebundenen Sauerstoffatom einen Ring bildet, worin ϕ einen Phenylrest darstellt; ein H-Atom oder einen Sulfonatrest darstellt;
X₄ ein H-Atom, einen Sulfonatrest oder das Kohlenstoffatom des -CHϕ-Restes, dargestellt durch X₃, darstellt;
R₁ einen Allylrest mit 2 bis 5 Kohlenstoffatomen oder einen Alkylrest mit 1 bis 5 Kohlenstoffatomen darstellt;
R₂ einen Alkylrest mit 1 bis 5 Kohlenstoffatomen darstellt;
Y₃ einen Benzylrest, ein H-Atom oder einen Sulfonatrest darstellt;
Y₅ ein H-Atom oder einen Sulfonatrest darstellt.
wonach:
- eine Verbindung der allgemeinen Formel (B) 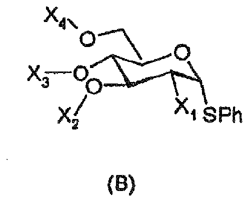
worin
X₁ einen Azidrest oder einen NHCOR₃-Rest, worin R₃ ein Alkyl- oder Arylrest mit 1 bis 5 Kohlenstoffatomen ist, darstellt;
X₂ einen Benzylrest, ein H-Atom oder einen Sulfonatrest darstellt;
X₃ einen -CH₂ϕ-Rest, einen -CHϕ-Rest, der mit dem an C(5) gebundenen Sauerstoffatom einen Ring bildet, worin ϕ einen Phenylrest darstellt; ein H-Atom oder einen Sulfonatrest darstellt;
X₄ ein H-Atom, einen Sulfonatrest oder das Kohlenstoffatom des -CHϕ-Restes, dargestellt durch X₃, darstellt;
Ph einen Phenylrest darstellt;
mit einer Verbindung der allgemeinen Formel (C) 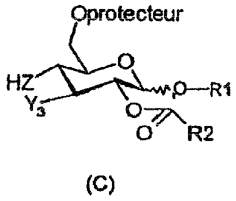 worin
Z ein O- oder S-Atom darstellt;
R₁ einen Allylrest mit 2 bis 5 Kohlenstoffatomen darstellt;
R₂ einen Alkylrest mit 1 bis 5 Kohlenstoffatomen darstellt;
Y₃ einen Benzylrest, ein Wasserstoffatom oder einen Sulfonatrest darstellt; protecteur eine Schutzgruppe darstellt;
umgesetzt wird, um eine Verbindung der allgemeinen Formel (D) zu erhalten 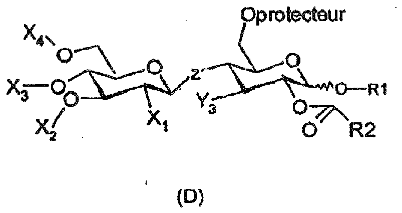 worin
Z ein β-ständiges O- oder S-Atom darstellt;
X₁ einen Azidrest oder einen NHCOR₃-Rest, worin R₃ ein Alkyl- oder Arylrest mit 1 bis 5 Kohlenstoffatomen ist, darstellt;
X₂ einen Benzylrest, ein H-Atom oder einen Sulfonatrest darstellt;
X₃ einen -CH₂ϕ-Rest oder einen -CHϕ-Rest darstellt, der mit dem an C(5) gebundenen Sauerstoffatom einen Ring bildet, worin ϕ einen Phenylrest darstellt; X₄ ein Wasserstoffatom, einen Sulfonatrest oder das Kohlenstoffatom des -CHϕ-Restes, dargestellt durch X₃, darstellt;
R₁ einen α- oder β-ständigen Allylrest mit 2 bis 5 Kohlenstoffatomen darstellt;
R₂ einen Alkylrest mit 1 bis 5 Kohlenstoffatomen darstellt;
Y₃ einen Benzylrest, ein Wasserstoffatom oder einen Sulfonatrest darstellt; protecteur eine Schutzgruppe darstellt;
- die Verbindung (D), welche eine β(1,4)-glycosidische Bindung aufweist, derart umgesetzt wird, dass die Schutzgruppe durch ein H-Atom oder einen Sulfonate rest substituiert wird, um die Verbindung der allgemeinen Formel (A) zu erhalten.

## Claims

1. Chemical compound of the disaccharide type, having the following general formula (A): 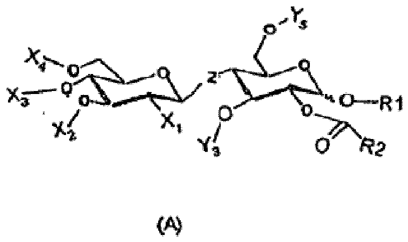 wherein
Z is an O or S atom in the β position;
X₁ is an azido group or an NHCOR₃ group, in which R₃ is an alkyl or aryl group containing 1 to 5 carbon atoms;
X₂ is a benzyl group, an H atom or a sulfonate group;
X₃ is a -CH₂ϕ group, a -CHϕ group forming a cycle with the oxygen atom at C(5), in which ϕ is a phenyl group, an H atom or a sulfonate group;
X₄ is an H atom, a sulfonate group or the carbon atom of the -CHϕ group represented by X₃;
R₁ is an allyl group containing 2 to 5 carbon atoms or an alkyl group containing 1 to 5 carbon atoms, the OR₁ bond being in the α or β position;
R₂ is an alkyl group containing 1 to 5 carbon atoms;
Y₃ is a benzyl group, an H atom or a sulfonate group;
Y₅ is an H atom or a sulfonate group.

2. Chemical compound according to claim 1, **characterised in that**:
Z is an O atom in the β position;
X₁ is an azido group;
X₂ is a benzyl group;
X₃ is a -CHϕ group forming a cycle with the oxygen atom at C(5), in which ϕ is a phenyl group;
X₄ is the carbon atom of the -CHϕ group represented by X₃;
R₁ is an allyl group containing 2 to 5 carbon atoms, the OR₁ bond being in the α or β position;
R₂ is a methyl group;
Y₃ is a benzyl group;
Y₅ is a hydrogen atom.

3. Chemical compound according to claim 1, **characterised in that**:
Z is an O atom in the β position;
X₁ is an NHCOR₃ group, in which R₃ is a methyl group;
X₂ is a benzyl group;
X₃ is a -CHϕ group forming a cycle with the oxygen atom at C(5), in which ϕ is a phenyl group;
X₄ is the carbon atom of the -CHϕ group represented by X₃;
R₁ is an allyl group containing 2 to 5 carbon atoms, the OR₁ bond being in the α or β position;
R₂ is a methyl group;
Y₃ is a benzyl group;
Y₅ is a hydrogen atom.

4. Chemical compound according to claim 1, **characterised in that**:
Z is an O atom in the β position;
X₁ is an NHCOR₃ group, in which R₃ is a methyl group;
X₂ is a benzyl group;
X₃ is a -CHϕ group forming a cycle with the oxygen atom at C₅, in which ϕ is a phenyl group;
X₄ is the carbon atom of the -CHϕ group represented by X₃;
R₁ is an allyl group containing 2 to 5 carbon atoms, the OR₁ bond being in the α or β position;
R₂ is a methyl group;
Y₃ is a benzyl group;
Y₅ is a sulfonate group.

5. Chemical compound according to claim 1, **characterised in that**:
Z is an O atom in the β position;
X₁ is an NHCOR₃ group, in which R₃ is a methyl group;
X₂ is an H atom;
X₃ is an H atom;
X₄ is an H atom;
R₁ is a propyl group, the OR₁ bond being in the α or β position;
R₂ is a methyl group;
Y₃ is an H atom;
Y₅ is an H atom.

6. Chemical compound according to claim 1, **characterised in that**:
Z is an O atom in the β position;
X₁ is an NHCOR₃ group, in which R₃ is a methyl group;
X₂ is an H atom;
X₃ is an H atom;
X₄ is an H atom;
R₁ is a propyl group, the OR₁ bond being in the α or β position;
R₂ is a methyl group;
Y₃ is an H atom;
Y₅ is a sulfonate group.

7. Composition comprising at least one compound according to any of claims 1 to 6, used as a medicine, a prophylactic agent or a phytosanitary agent.

8. Composition comprising at least one compound according to any of claims 1 to 6, used as a chitinase inhibitor.

9. Composition comprising at least one compound according to any of claims 1 to 6, used as an insecticide.

10. Composition according to any of claims 7 to 9, **characterised in that** it contains a concentration of said compound or compounds of between 10 and 100 µg/ml, and preferably of between 10 and 50 µg/ml.

11. Method for preparing a compound having the general formula (A), 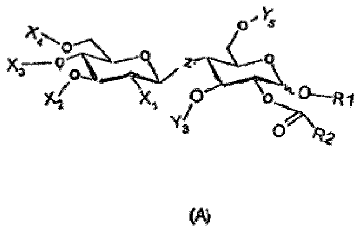 wherein Z is an O or S atom in the β position;
X₁ is an azido group or an NHCOR₃ group, in which R₃ is an alkyl or aryl group containing 1 to 5 carbon atoms;
X₂ is a benzyl group, an H atom or a sulfonate group;
X₃ is a -CH₂ϕ group, a -CHϕ group forming a cycle with the oxygen atom at C(5), in which ϕ is a phenyl group, an H atom or a sulfonate group;
X₄ is an H atom, a sulfonate group or the carbon atom of the -CHϕ group represented by X₃; R₁ is an allyl group containing 2 to 5 carbon atoms or an alkyl group containing 1 to 5 carbon atoms;
R₂ is an alkyl group containing 1 to 5 carbon atoms;
Y₃ is a benzyl group, an H atom or a sulfonate group;
Y₅ is an H atom or a sulfonate group,
according to which method
- a compound having the general formula (B), 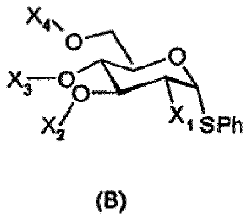
wherein
X₁ is an azido group or an NHCOR₃ group, in which R₃ is an alkyl or aryl group containing 1 to 5 carbon atoms;
X₂ is a benzyl group, an H atom or a sulfonate group;
X₃ is a -CH₂ϕ group, a -CHϕ group forming a cycle with the oxygen atom at C(5), in which ϕ is a phenyl group, an H atom or a sulfonate group;
X₄ is an H atom, a sulfonate group or the carbon atom of the -CHϕ group represented by X₃; Ph is a phenyl group;
is reacted with a compound having the general formula (C), 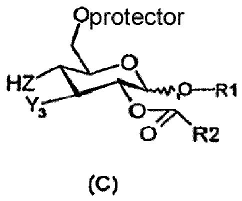 wherein
Z is an O or S atom;
R₁ is an allyl group containing 2 to 5 carbon atoms;
R₂ is an alkyl group containing 1 to 5 carbon atoms;
Y₃ is a benzyl group, a hydrogen atom or a sulfonate group; protector is a protector group;
in order to obtain a compound having the general formula (D), 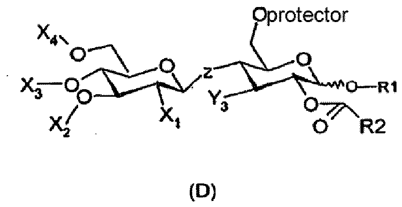 wherein
Z is an O or S atom in the β position;
X₁ is an azido group or an NHCOR₃ group, in which R₃ is an alkyl or aryl group containing 1 to 5 carbon atoms;
X₂ is a benzyl group, an H atom or a sulfonate group;
X₃ is a -CH₂ϕ group or a -CHϕ group forming a cycle with the oxygen atom at C(5), in which ϕ is a phenyl group;
X₄ is a hydrogen atom, a sulfonate group or the carbon atom of the -CHϕ group represented by X₃;
R₁ is an allyl group in the α or β position containing 2 to 5 carbon atoms;
R₂ is an alkyl group containing 1 to 5 carbon atoms;
Y₃ is a benzyl group, a hydrogen atom or a sulfonate group;
protector is a protector group;
- said compound (D) having a β (1, 4) glycosidic bond is reacted so as to substitute the protector group with an H atom or a sulfonate group in order to obtain said compound having the general formula (A).
